# EUROPEAN PATENT SPECIFICATION

(11) **EP 1 898 977 B1**
(45) Date of publication and mention of the grant of the patent: **14.04.2010**
(21) Application number: 06747999.8
(22) Date of filing: 24.05.2006
(51) Int. Cl.: A61M 15/00

(54) **DOSE COUNTER**
DOSISZÄHLER
COMPTEUR DE DOSES

(30) Priority: 24.05.2005 US 683778 P; 24.05.2005 EP 05104409; 20.12.2005 SE 0502812
(43) Date of publication of application: 19.03.2008
(73) Proprietor: Letcat Aktiebolag, 223 69 Lund (SE)
(72) Inventor: BRUNNBERG, Lennart, S-135 49 Tyresö (SE); LANDAHL, Henrik, S-227 63 Lund (SE)
(74) Representative: Bergström, Johan Erik
(86) International application number: PCT/SE2006/050159
(87) International publication number: WO 2006/126967

(56) References cited:
- WO-A-01/93932
- GB-A- 2 398 065
- GB-A- 2 398 065
- US-A1- 2004 069 301
- US-A1- 2005 028 815

## Description

### TECHNICAL FIELD

The invention refers to a dose counter device for an inhaler that in a reliable way will register a delivered dose from a canister comprised in the inhaler, and that at the same time substantially will reduce the risk of falsely register a dose not delivered. Thus, the present invention will in an effective way, substantially avoid miscalculations of delivered doses from the canister.

### BACKGROUND OF THE INVENTION

Within the field of inhalers, dose counters are known that will count the number of doses delivered from a canister comprised in the inhaler. The user will thus for instance know, the number of doses taken or the numbers of doses remaining in the canister. A problem with known dose counters, is that they at times will register a delivered dose that never was delivered, and that they also may miss to register a dose that in fact was delivered. The user of the inhaler is thus provided with false information about the number of doses remaining in the inhaler, which may constitute a major problem for for instance an asthmatic person which thus unintentionally may run out of medicament.

A few solutions of mechanical dose counters have been presented during a number of years, such as for example EP-A2-0966309, wherein a dose counter is located near the valve region of the canister and attached to the base of an actuator, wherein the displacement of the top of the canister relative to the valve stem is measured.

In EP-A1-0254391, a dose counter is located on the top of the inhalation device, wherein the displacement of the top of the canister relative the actuator body is measured.

Since canisters suffer from manufacturing height dimension variations and the counters in EP-A2-0966309 and in EP-A1-0254391 work taking into account the displacement of the canister, there exists a great risk of having counting errors.

There has also been presented a number of electrical and/or electronic solutions of dose counters. For example, GB 2288259 discloses a dose counter that comprises a button having a piezo-electric film sensor sensing the force applied to the button and the top of the canister when this is to be depressed for delivering a dose. When the force has reached a certain threshold value, a signal is sent to the electronic counter which registers a delivered dose.

GB 2398065 discloses a solution where an acoustic sensor in the form of a piezo-electric film is arranged on the canister at the outlet stem. The sensor registers the vibrations in the canister when a dose is delivered. The signal from the sensor is sent to a circuit at the top of the canister which registers a delivered dose.

The drawback with the solution according to GB 2288259 is that force measurement requires that the forces required to actually deliver a dose are held within a quite narrow range. The forces required depend on the spring in the canister and friction when pushing the stem into the canister, which can vary within a large range. This in turn means that the force sensed by the sensor may not be enough to deliver a dose so that the dose counter counts a dose even if none is delivered, or that the force required to deliver a dose is lower than the force level that is registered.

The drawback with the solution according to GB 2398065 is that the sensor is placed inside the inhaler close to the canister stem and that wiring is required between the sensor and the circuit at the top of the canister. It is thus rather difficult to arrange the dose counter to the canister, which could be done by an inexperienced user. If the sensor is not attached properly, the delivered doses will not be registered in a proper way, which in turn would lead to deviations between delivered number of doses and registered number of doses. Further, the placing of components inside the inhaler and thus in the inhalation airflow may affect the function of the inhaler in a negative way.

Document WO 01/93932 discloses a medicament delivery device in the form of an inhaler adapted to handle aerosol driven medicament containers that provide a metered dose of medicament as aerosol in a mouth piece when the canister is depressed.

The device further comprises a dose counter mechanism positioned to sense when the canister has been reciprocated within the housing of the device. The sensing means could be mechanical, optical, electrical or the like.

In view of the above, there is a general need for a dose counter device that in a reliable way will register a delivered dose from a canister comprised in the inhaler, and that at the same time substantially will reduce the risk of falsely register a dose not delivered. As understood, it is very difficult, if not impossible; to design a device that registers the in fact delivered dose with 100% accuracy. However, from the users' point of view, it is better to have at hand a dose counter device that occasionally may register a dose delivery even though no dose was in fact delivered, than to have it the other way around. In this way, the user can not unintentionally run out of medicament.

### BRIEF DESCRIPTION OF THE INVENTION

The aim of the present invention is to remedy the above mentioned problems with accurate dose counters that are applicable to standard inhalers and standard canisters having differences in tolerances.

This aim is solved by an inhaler according to claim 1. Preferable embodiments of the invention are subject to the dependent claims.

According to a main aspect of the invention, it is characterised by a dose counter device for an inhaler that registers when a dose is delivered, adapted to be mounted on the distal end of the inhaler, said inhaler comprises a canister comprised in an inhaler housing, wherein the distal end of the canister protrudes a distance from the distal end of the inhaler housing, and wherein the canister has a canister body and further in its proximal end has a dose chamber having a valve means and a transfer tube having a valve means, wherein the valve means are placed a predetermined distance from each other along the longitudinal axis of the canister when the inhaler is in a first non-activated state, and wherein the valves are adapted to communicate with each other when the distal end of the canister in a second activated state is applied with a force that urges the canister chamber over the transfer tube towards the proximal end of the canister a distance that is equal to said predetermined distance, which will expel medicament from the canister, characterised in that the dose counter device comprises device trigger means provided in an electronic circuit on the distal end of the canister, that the electronic circuit further comprises an acoustic sensing means also provided on the distal end of the canister, and in that the device trigger means is adapted to activate the acoustic sensing means when a force is applied to the distal end, of the canister is substantially equal to and/or above a predetermined force value, and in that the acoustic sensing means registers a dose delivery when it picks up a sound.

According to another aspect of the invention, it is characterised in that the device trigger means is a contact and that the acoustic sensing means is activated when the contact is closed due to the force applied to the distal end of the canister.

According to yet an aspect of the invention it is characterised in that the device trigger means is a force sensing means, and that said force sensing means is adapted to determine the force value applied to the distal end of the canister during the second activated state of the inhaler, and that the acoustic sensing means is activated when the force that is applied to the distal end of the canister is substantially equal to and/or above a predetermined force value,

According to another aspect of the invention it is characterised in that the force sensing means is a strain gauge or a piezo electric element.

According to a further aspect of the invention, it is characterised in that the device trigger means comprises both a contact and a force sensing means, and that the closing of the contact activates the force sensing means.

According to a further aspect of the invention, it is characterised in that the acoustic sensing means is a piezo electric element, a strain gauge or a microphone or the like.

According to yet an aspect of the invention, it is characterised in that the force sensing means is also used as the acoustic sensing means.

According to a further aspect of the invention it is characterised in that the acoustic sensing means is provided with means that compares the spectra of a picked up sound with the spectra of the characteristic sound of a delivered dose and if there is a match between said spectra will register a sound as a dose delivery.

The present invention has a number of advantages compared to the state of the art devices. One obvious advantage is that both the device trigger means and the acoustic sensing means are placed at the distal end of the canister and thus the device. This means that it is easy for example to attach to the distal end of a canister of a press-and-breath inhaler and to use the device without having to adapt the inhaler to the device and/or to have components that have to be arranged inside the inhaler, which could be difficult for a patient to accomplish. Further, because no components need to be arranged in the interior of the inhaler, the device or its components will thus not affect the function or airflows through the device during inhalation. This may have the additional advantage that inhalers that have been approved by governmental authorities, such as the American FDA, do not need a further approval. The device is further easy and uncomplicated to manufacture,

Another advantage is that the acoustic sensing means is only activated or triggered when a force is applied to the canister, i.e. when a dose is to be delivered. This means that the acoustic sensing means cannot unintentionally register a sound, which may not be the sound of a delivered dose, i.e. it listens only during the time of dose delivery. Also, this greatly reduces the power consumption of the device.

One advantage when the force sensing means and the acoustic sensing means are one and the same component, is that the number of components are reduced and thereby the manufacturing cost of the device is reduced. When a piezo electric element is used, it has the advantage that the power consumption is very low, and also that the device can be made very compact. Because of the properties of the piezo electric element, it may be used for other features such as sound generating, for example alerting a user that it is time to take a dose or to warn the user that there are only a few doses left in the canister.

The device could also have a "learning" ability, that it registers the sound spectra of delivered doses from a certain canister and then compares the sensed sound with the registered spectra. In that way the risk of wrongly sensing and registering sounds are further reduced. The learning ability could for example be done during the initial doses that are fired when a new canister is to be used. The learning ability means that the acoustic sensing means will function with any type of canister regardless of substance, choice of material of the canister and the mechanics. Because of the learning ability and adaptive function the acoustic sensing means will handle any possible change of the sound of a delivered dose during the life of the canister, which change of sound for example may be due to wear of components of the canister. It is also possible to have a rough "basic" reference spectra of a typical sound of a delivered dose stored in the electronic circuitry of the device, which basic spectra is used as the "starting" spectra for the acoustic sensing means to detect a delivered dose. During the initially fired doses the electronic circuitry modifies the basic spectra to the actual detected spectra.

In order to have a "double" security against unintended activation, the device trigger means could comprise both a contact and a force sensing means, such as a piezo electric element and arranged such that the force sensing means is only activated when the contact breaker is closed, which is done when canister begins to be depressed. The closing of the contact also activates the rest of the circuitry of the device.

These and other aspects of and advantages with the present invention will become apparent from the following detailed description and from the accompanying drawings.

### BRIEF DESCRIPTION OF THE DRAWINGS

In the following detailed description of the invention, reference will be made to the accompanying drawings, of which
Fig. 1 illustrates a general inhaler comprising a liquid medicament containing canister, when the inhaler is in a non-activated state,
Fig. 2 shows a cross-section of an electronic dose counter device to be used with a medical dispenser,
Fig. 3 shows a schematic block diagram of the dose counter of Fig. 2.

### DETAILED DESCRIPTION OF THE INVENTION

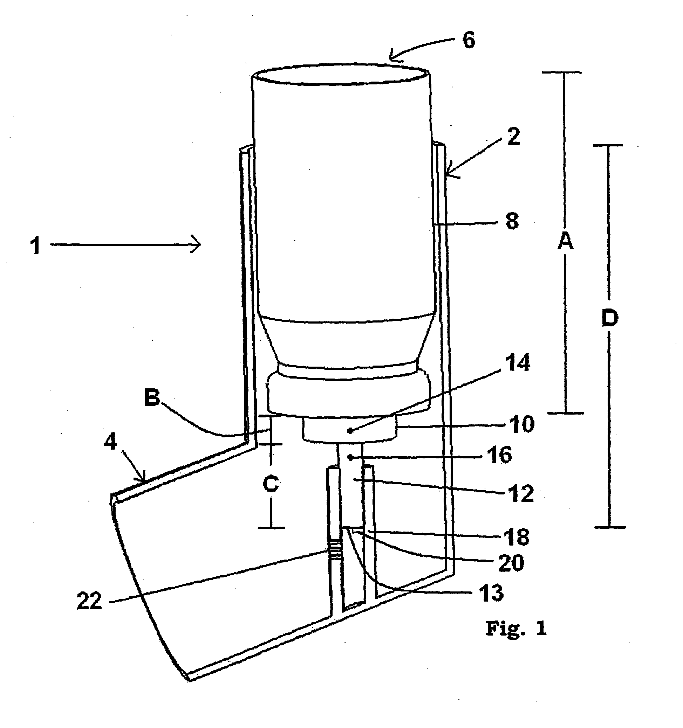

With reference to figure 1, a general inhaler 1 comprises a housing 2 having a mouthpiece 4, which the user puts in his mouth when a dose of medicament is to be inhaled. The housing 2 of the inhaler is adapted to receive a standard canister 6, containing liquid medicament, wherein the distal end of the canister 6 protrude a certain distance from the distal end of the housing 2.

The canister comprises a main canister body 8 that is adapted to communicate with a dose chamber 10. The dose chamber 10 is in turn provided with a hollow spring-suspended transfer tube 12 provided with an outlet 13 in its proximal end. The dose chamber is further provided with an outlet valve 14 that is adapted to correspond to a valve 16 in the transfer tube 12.

The interior of the mouthpiece 4 is provided with a tubular receiving member 18, having an inward protruding flange 20, provided a predetermined distance from the bottom of the receiving member 18. The receiving member 18 is further provided with an outlet 22 that communicates with the outlet of the mouthpiece 4.

The proximal end of the transfer tube 12 abuts against the flange 20, such that a part of the outlet 13 of the transfer tube communicates with the outlet 22 of the receiving member.

When a user of the inhaler intends to inhale a dose, he puts the mouthpiece in his mouth and applies a force, generally by the aid of his hand and fingers or the like, on the distal surface of the canister 6, such that the canister body 8 and the dose chamber 10 is forced downwards towards the bottom of the housing 2, i.e. towards the proximal end of the canister, while the transfer tube 12 remains still. Thus, when the dose chamber 10 has moved a predetermined distance towards the bottom of the housing, the valve 16 of the transfer tube 12 will open communication with the valve 14 of the dose chamber, such that a metered dose of the pressurised liquid medicament contained in the main body 8 will flow from the dose chamber 10, through the valves 14, 16, the transfer tube 12, the outlets 13, 22 and out through the outlet of the mouthpiece 4. When the user releases the force applied to the distal end of the canister, it will move back to its original position.

The distance between the valves 14, 16 is a known predetermined distance, generally 2 mm, when the inhaler is in the first non activated state, i.e. the dose chamber needs to in a second activated state be forced downwards with a distance of 2 mm for the valves 14, 16 to open communication with each other.

It would be a simple case to design a reliable dose counter device if the dimensions of the inhaler, would be exact dimensions. However, all dimensions of the general inhaler, such as the height of the main body A, the height of the dose chamber B, the length of the transfer tube C, and the distance between the proximal end of the transfer tube and the distal edge of the housing D, are impaired by variation of not negligible magnitude. If for instance the variation of the distances A, B, C and D is ±0.5 mm, ±0.05 mm, ±0.25 mm and ±0.2 mm, respectively, the sum of all margin of errors will be ± 1 mm.

So, if one for instance designs a dose counter device that determines the distance that the canister has moved towards the bottom of the housing, dependent on a single reference point, for instance the distal edge of the housing 2, and register a delivered dose when said distance amount to 2 mm, the dose may or may not have been delivered. Also, the dose may have been delivered without the distance amounting to 2 mm.

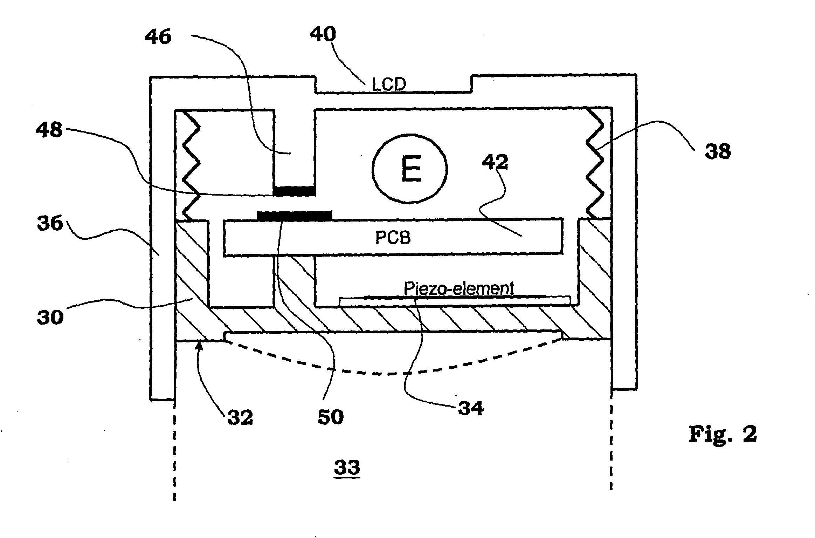

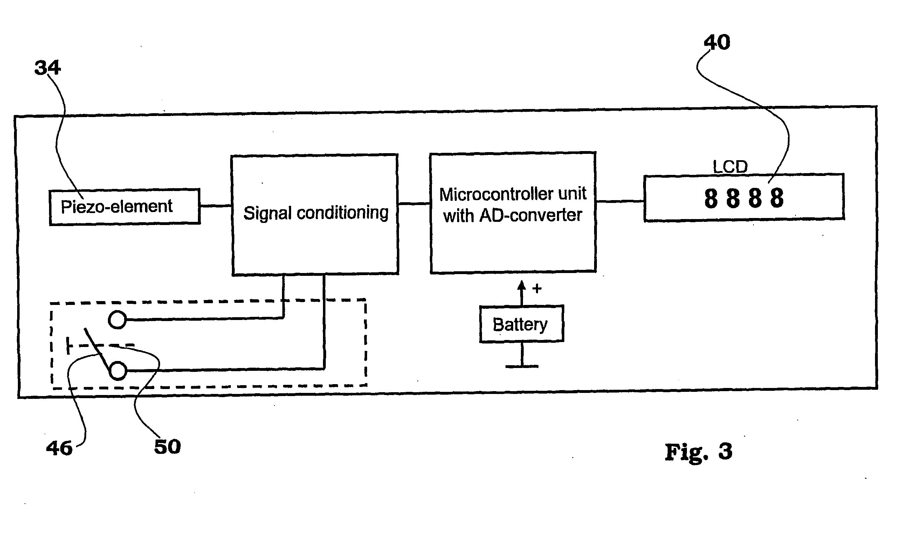

According to the present invention, a dose counter is presented, which is not at all dependent on the differences in tolerances of the inhaler. The dose counter, Figs. 2 and 3, comprises a bottom enclosure 30 having a bottom surface 32 with a shape that generally corresponds to the upper surface of the canister 33. Attachment means such as glue, double adhesive tape or the like is provided for attaching the bottom enclosure to the outer area of the upper surface of the canister. The attachment surface of the bottom enclosure could be provided with a circumferential ledges shown in Fig. 2. In the enclosure a piezo-electric element 34 is attached to the upper bottom surface.

Surrounding the bottom enclosure a top enclosure 36 is arranged, which is movable in the vertical direction of the device and the bottom enclosure 30 against the force of a spring 38. On the top of the top enclosure a display 40 is arranged, for example an LCD display. This in turn is connected to a printed circuit board 42 comprising an electronic circuitry which may for example comprise processors, and I/O means and other applicable components for handling the function of the device according to the invention, as will be described in detail below. The top enclosure is further arranged with a downwardly protruding arm 46 or protrusion.

The piezo electric element acts as a device trigger means and may also have a double function as realized below, both as a force sensing means and an acoustic sensing means. When the patient or user depresses the top enclosure for delivering a dose of medicament the protrusion 46 will apply a pressure or force on the bottom enclosure 30. this force will cause the central part of the bottom wall of the bottom enclosure to bend due to that the bottom enclosure is only in contact with the canister at its periphery. The bending of the bottom enclosure will affect the piezo electric element, and a voltage is generated in the piezo electric element. When the force value amounts to a certain value, for instance 10 N that in many cases and for many canisters corresponds to a movement of the canister body towards the proximal end of the canister with a distance of 1 mm, will activate the electronic circuit in that an electric voltage is generated by the piezo electric element. At this moment the electronic circuit will start to sample signals from the piezo electric element in that the element now acts as an acoustic sensing means, i.e. the elements "listens" for sound. The delivery of a dose from the canister will namely generate a sound that will propagate to the distal end of the canister and which can be registered by the piezo electric element as a delivered dose.

By the above solution the piezo electric element is only activated to listen and detect the sound of a delivered dose when a force is applied to the piezo electric element, for example above or equal to 10 N, i.e. The acoustic sensing means is adapted to operate within a predetermined range. This will minimize the risk of the acoustic sensing means registering a sound that is not originating from the delivery of a dose. When the force applied by the user amounts to a certain force, one can be certain that the user intends to inhale a dose and that the sound of the dose delivery is soon to follow. Moreover, when the inhaler is not in use, the electronic circuit is not closed and thus the acoustic sensing means is not active. However the display is preferably activated so that the patient at all times can view the number of doses. However, very little energy is consumed.

Preferably the electronic circuit is provided with means, such as a signal interpreting means, so that the acoustic sensing means is adapted to register a sound as a delivered dose, only when it picks up a sound that corresponds to the sound that has its origin from the delivery of a dose. The delivery of a dose from the canister, namely generates a characteristic sound that can be identifiable by means of the signal interpreting means. Said interpreting means can for instance be provided with means that compares the spectra of the picked up sound with the spectra of the sound of a delivered dose. If there is a mismatch between said spectra, a sound is not registered as a dose delivery since the sound picked up thus had its origin from something else.

In order to further minimize the power need of the dose counter device, said device can be provided with an additional device trigger means that closes the electronic circuit provided on the distal end of the canister only when a certain force is applied to said canister. This could for example, as shown in Fig. 2 be a conducting surface 48 arranged on the end of the protrusion 46, which conducting surface, when the top enclosure is depressed and the protrusion comes in contact with a contact surface 50 on the bottom enclosure, closes a circuit which activates the electronic circuit of the device. Only after closing the contact, the force sensing means will be activated. The closing of the contact also activates the rest of the circuitry of the device.

The dose counter device is preferably provided with means in order to be connected to an external power source, such as a battery, even though it might be possible for the piezo electric element to be the only power source needed to operate the device.

It is of course feasible to use other components instead of a piezo electric element. For example a strain gauge could be used as the force sensing means. In a simplified variant of the device, the device trigger means could be just a contact and when the contact is closed, this activates the acoustic sensing means to start to listen for the specific sound of a delivered dose. It is also conceivable that the acoustic sensing means is a microphone and the like component that is capable of registering specific sound or vibration spectra, also spectra outside the audible spectra.

A further conceivable development of the device is to use "micro-mechanics", i.e. to integrate several electrical and mechanical components in one or more chips, like for example acoustic sensing means, force sensing means and other types of components and functions on a miniature bases.

Preferably the force sensing means is also used as the acoustic sensing means. For instance, if a strain gauge is used as the force sensing means, a at least one further strain gauge can be provided on the distal end of the canister, which strain gauge is used as the acoustic sensing means as described above. It is also feasible that the strain gauge that serves as the force sensing means, is adapted to also have the function of being the acoustic sensing means. The same situation applies when the force sensing means is a piezo electric element. That is, the piezo electric element used as the force sensing means can also be used as the acoustic sensing means, or at least one further piezo electric element, provided on the distal end of the canister, can be used as the acoustic sensing means. Naturally, the use of a strain gauge as the force sensing means does not rule out the use of a piezo electric element as the acoustic sensing means, and vice versa. The components can thus be used in any combination. If a piezo electric element is used as the force sensing means and/or the acoustic sensing means, the dose counter device can be provided with spring means in order to reduce the flex of the piezo electric element in order to reduce of the risk for said element to break or crack.

The delivered dose and/or the doses remaining in the canister can be visualized for the user in a number of ways, such as through an electronic display provided in the inhaler or the like. Information about taken or remaining doses and e.g. time point may also be distributed by e.g. radiofrequency such as Bluetooth to another device where the information is displayed or used for compliance measuring, as described for instance in SE0300729-1.

Moreover, when a piezo electric element is used, because of its properties, it can be used to produce sound for example to alert a user at certain time intervals to take a dose of medicament or to warn the user that for example only ten doses remain and that the user soon should replace the canister with a new. In that aspect the circuit is pre-programmed with the total number of doses in a canister and when registering delivered doses counts down and displays the remaining number of doses. According to another aspect of the invention, the electronic circuit could be configured so that the registered sounds from the canister when the first two or three doses are delivered are stored and compared in order for the circuit to "learn" the specific spectrum of that canister, in order to increase the reliability that only the sounds of a delivered dose is registered.

The electronic circuit could also be provided with additional means for registering and monitoring the delivery of doses. For example, the electronic circuit could be provided with temperature sensors for measuring and storing the actual temperature at the time a dose was delivered. The circuitry then has to be added with a real-time clock for keeping track of time. It may also be provided with means for detecting and monitoring the air flow during inhalation, for measuring and storing the air flows at dose delivery. As a conceivable solution, the acoustic sensing means could also be able to listen to the specific sounds connected to inhalation. In that aspect, the learning function could be used as well as the comparison between the detected spectra and previously stored spectres.

Further, accelerometers could be provided for acting as shaking sensors for registering if the device has been shaken before use. All the information from these features could be used to register if the patient has been able to receive the doses properly, how the conditions during dose delivery were, i.e. to obtain a dose delivery history, so that a physician can advice its patient, and to maybe change the behaviour of the patient regarding handling of the device, change the frequency of delivered doses and the like.

It is to be understood that the embodiments described above and shown in the drawings are to be regarded only as non-limiting examples of the invention and that it may be modified in many ways within the scope of the patent claims.

## Claims

1. Dose counter device for an inhaler that registers when a dose is delivered, adapted to be mounted on the distal end of the inhaler (1), said inhaler (1) comprises a canister (6) comprised in an inhaler housing (2), wherein the distal end of the canister (6) protrudes a distance from the distal end of the inhaler housing (2), and wherein the canister (6) has a canister body (8) and further in its proximal end has a dose chamber (10) having a valve means (14) and a transfer tube (12) having a valve means (16), wherein the valve means (14; 16) are placed a predetermined distance from each other along the longitudinal axis of the canister (6) when the inhaler (1) is in a first non-activated state, and wherein the valves (14; 16) are adapted to communicate with each other when the distal end of the canister (6) in a second activated state is applied with a force that urges the canister chamber (10) over the transfer tube (12) towards the proximal end of the canister (6) a distance that is equal to said predetermined distance, which will expel medicament from the canister (6), device trigger means (34, 48, 50) provided in an electronic circuit on the distal end of the canister, **characterised in that** the electronic circuit further comprises an acoustic sensing means (34) also provided on the distal end of the canister, and **in that** the device trigger means (34, 48, 50) is adapted to activate the acoustic sensing means (34) when a force is applied to the distal end of the canister (6) and **in that** the acoustic sensing means (34) registers a dose delivery when it picks up a sound.

2. Dose counter device according to claim 1, **characterised in that** the device trigger means is a contact (48, 50) and that the acoustic sensing means (34) is activated when the contact (48, 50) is closed due to the force applied to the distal end of the canister.

3. Dose counter device according to claim 1, **characterised in that** the device trigger means (34) is a force sensing means (34), and that said force sensing means is adapted to determine the force value applied to the distal end of the canister during the second activated state of the inhaler, and that the acoustic sensing means (34) is activated when the force that is applied to the distal end of the canister is substantially equal to and/or above a predetermined force value,

4. Dose counter device according to claim 3, **characterised in that** the force sensing means (34) is a strain gauge or a piezo electric element.

5. Dose counter device according to claims 2-3, **characterised in that** the device trigger means comprises both a contact (48, 50) and a force sensing means (34), and that the closing of the contact activates the force sensing means.

6. Dose counter device according to claim 1, **characterised in that** the acoustic sensing means is a piezo electric element, a strain gauge or a microphone, or the like.

7. Dose counter device according to any of the preceding claims, **characterised in that** the force sensing means is also used as the acoustic sensing means.

8. Dose counter device according to any of the preceding claims, **characterised in that** the acoustic sensing means is provided with means that compares the spectra of a picked up sound with the spectra of the characteristic sound of a delivered dose and if there is a match between said spectra will register a sound as a dose delivery.

## Patentansprüche

1. Dosiszähler für einen Inhalator, wobei der Dosiszähler registriert, wenn eine Dosis abgegeben wurde, und dazu geeignet ist, am distalen Ende des Inhalators (1) montiert zu werden, wobei der Inhalator (1) aufweist:
einen Behälter (6), der in einem Inhalatorgehäuse (2) aufgenommen ist, wobei das distale Ende des Behälters (6) über einen Abstand vom distalen Ende des Inhalatorgehäuses (2) hervorsteht, und wobei der Behälter (6) einen Behälterkörper (8) aufweist und ferner an seinem proximalen Ende eine Dosiskammer (10) mit einem Ventil (14) und ein Übertragungsrohr (12) mit einem Ventil (16) aufweist, wobei die Ventile (14; 16) entlang der Längsachse des Behälters (6) in einem vorgegebenen Abstand voneinander angeordnet sind, wenn der Inhalator (1) sich in einem ersten, nicht aktivierten Zustand befindet, und wobei die Ventile (14; 16) dazu geeignet sind, miteinander zu kommunizieren, wenn auf das distale Ende des Behälters (6) in einem zweiten, aktivierten Zustand eine Kraft ausgeübt wird, die die Behälterkammer (10) über dem Übertragungsrohr (12) über eine Strecke zum proximalen Ende des Behälters (10) hin zwingt, die dem vorgegebenen Abstand gleicht, wodurch ein Medikament vom Behälter (6) ausgegeben wird; und
eine in einer elektronischen Schaltung am distalen Ende des Behälters angeordnete Triggereinrichtung (34, 48, 50);
**dadurch gekennzeichnet, dass**
die elektronische Schaltung ferner einen Schallsensor (34) aufweist, der ebenfalls am distalen Ende des Behälters angeordnet ist,
die Triggereinrichtung (34, 48, 50) dazu geeignet ist, den Schallsensor (34) zu aktivieren, wenn eine Kraft auf das distale Ende des Behälters (6) ausgeübt wird, und
der Schallsensor (34) eine Dosisabgabe registriert, wenn er ein Geräusch erfasst.

2. Dosiszähler nach Anspruch 1,
**dadurch gekennzeichnet, dass**
die Triggereinrichtung ein Kontakt (48, 50) ist, und
der Schallsensor (34) aktiviert wird, wenn der Kontakt (48, 50) durch die auf das distale Ende des Behälters ausgeübte Kraft geschlossen wird.

3. Dosiszähler nach Anspruch 1,
**dadurch gekennzeichnet, dass**
die Triggereinrichtung (34) ein Kraftsensor (34) ist,
der Kraftsensor dazu geeignet ist, die Größe der während des zweiten aktivierten Zustands des Inhalators auf das distale Ende des Behälters ausgeübten Kraft zu bestimmen, und
der Schallsensor (34) aktiviert wird, wenn die auf das distale Ende des Behälters ausgeübte Kraft einer vorgegebenen Kraft im Wesentlichen gleicht und/oder größer ist als die vorgegebene Kraft.

4. Dosiszähler nach Anspruch 3, **dadurch gekennzeichnet, dass** der Kraftsensor (34) ein Dehnungsmessstreifen oder ein piezoelektrisches Element ist.

5. Dosiszähler nach Anspruch 2 oder 3,
**dadurch gekennzeichnet, dass**
die Triggereinrichtung sowohl einen Kontakt (48, 50), als auch einen Kraftsensor (34) aufweist, und
der Kraftsensor durch Schließen des Kontakts aktiviert wird.

6. Dosiszähler nach Anspruch 1, **dadurch gekennzeichnet, dass** der Schallsensor ein piezoelektrisches Element, ein Dehnungsmessstreifen, ein Mikrofon oder eine ähnliche Einrichtung ist.

7. Dosiszähler nach einem der vorangehenden Ansprüche, **dadurch gekennzeichnet, dass** der Kraftsensor auch als Schallsensor verwendet wird.

8. Dosiszähler nach einem der vorangehenden Ansprüche, **dadurch gekennzeichnet, dass** der Schallsensor außerdem eine Einrichtung aufweist, die das Spektrum eines erfassten Geräuschs mit dem Spektrum eines charakteristischen Geräuschs einer abgegebenen Dosis vergleicht, wobei der Dosiszähler, wenn beide Spektren übereinstimmen, ein Geräusch als Dosisabgabe registriert.

## Revendications

1. Dispositif compteur de doses pour un inhalateur qui enregistre quand une dose est délivrée, adapté pour être monté sur l'extrémité distale de l'inhalateur (1), ledit inhalateur (1) comprend une cartouche (6) contenue dans un logement (2) d'inhalateur, l'extrémité distale de la cartouche (6) faisant saillie sur une distance de l'extrémité distale du logement (2) d'inhalateur, et la cartouche (6) ayant un corps (8) de cartouche et comprenant en outre à son extrémité proximale a une chambre à dose (10) ayant une moyen (14) formant soupape et un tube de transfert (12) ayant une moyen (16) formant soupape, les moyens (14, 16) formant soupape étant placés à une distance prédéterminée les uns des autres suivant l'axe longitudinal de la cartouche (6) lorsque l'inhalateur (1) est dans un premier état de non-activation, et les moyens (14, 16) formant soupape étant adaptés pour communiquer l'un avec l'autre lorsque l'extrémité distale de la cartouche (6), dans un second état d'activation, est appliquée avec une force qui pousse la chambre (10) de cartouche sur le tube de transfert (12) vers l'extrémité proximale de la cartouche (6) sur une distance qui est égale à ladite distance prédéterminée, ce qui va expulser du médicament hors de la cartouche (6), un moyen (34, 48, 50) de déclenchement du dispositif étant prévu dans un circuit électronique sur l'extrémité distale de la cartouche, **caractérisé en ce que** le circuit électronique comporte en outre un moyen de détection acoustique (34) également prévu sur l'extrémité distale de la cartouche, et **en ce que** le moyen (34, 48, 50) de déclenchement du dispositif est adapté pour activer le moyen de détection acoustique (34) lorsqu'une force est appliquée à l'extrémité distale de la cartouche (6) et **en ce que** le moyen de détection acoustique (34) enregistre une délivrance de dose lorsqu'il capte un son.

2. Dispositif compteur de doses selon la revendication 1, **caractérisé en ce que** le moyen (34, 48, 50) de déclenchement du dispositif est un contact (48, 50) et **en ce que** le moyen de détection acoustique (34) est activé lorsque le contact (48, 50) est fermé en raison de la force appliquée à l'extrémité distale de la cartouche.

3. Dispositif compteur de doses selon la revendication 1, **caractérisé en ce que** le moyen (34) de déclenchement du dispositif est un moyen (34) formant capteur de force, et **en ce que** ledit moyen formant capteur de force est adapté pour déterminer la valeur de la force appliquée à l'extrémité distale de la cartouche lors du deuxième état activé de l'inhalateur, et **en ce que** le moyen (34) de détection acoustique est activé lorsque la force qui est appliquée à l'extrémité distale de la cartouche est sensiblement égale à, et/ou au-dessus, d'une valeur de force prédéterminée.

4. Dispositif compteur de doses selon la revendication 3, **caractérisé en ce que** le moyen (34) formant capteur de force est une jauge de contrainte ou un élément piézo-électrique.

5. Dispositif compteur de doses selon les revendications 2 et 3, **caractérisé en ce que** le moyen de déclenchement du dispositif comprend à la fois un contact (48, 50) et un moyen (34) formant capteur de force, et **en ce que** la fermeture du contact active le moyen formant capteur de force.

6. Dispositif compteur de doses selon la revendication 1, **caractérisé en ce que** le moyen de détection acoustique est un élément piézo-électrique, une jauge de contrainte ou un microphone, ou similaires.

7. Dispositif compteur de doses selon l'une quelconque des revendications précédentes, **caractérisé en ce que** le moyen formant capteur de force est également utilisé en tant que le moyen de détection acoustique.

8. Dispositif compteur de doses selon l'une quelconque des revendications précédentes, **caractérisé en ce que** le moyen de détection acoustique est muni d'un moyen qui compare le spectre d'un son capté avec le spectre du son caractéristique d'une dose délivrée et s'il y a une correspondance entre lesdits spectres, enregistre un son comme étant une délivrance de dose.
